# EUROPEAN PATENT SPECIFICATION

(11) **EP 2 227 221 B1**
(45) Date of publication and mention of the grant of the patent: **13.02.2013**
(21) Application number: 08856371.3
(22) Date of filing: 05.12.2008
(51) Int. Cl.: A61K 9/00, A61K 31/205

(54) **EFFERVESCENT TABLETS AND EFFERVESCENT GRANULES CONTAINING CARNITINE TARTRATE**
CARNITINTARTRAT ENTHALTENDE BRAUSETABLETTEN UND BRAUSEGRANULAT
COMPRIMÉS EFFERVESCENTS ET GRANULÉS EFFERVESCENTS CONTENANT DU TARTRATE DE CARNITINE

(30) Priority: 07.12.2007 GR 20070100742
(43) Date of publication of application: 15.09.2010
(73) Proprietor: Uni-Pharma Kleon Tsetis Pharmaceutical Laboratories S.A., 145 64 Kifissia (GR)
(72) Inventor: TSETIS, Kleon, 145 64 Kifissia (GR)
(74) Representative: Wibbelmann, Jobst
(86) International application number: PCT/GR2008/000069
(87) International publication number: WO 2009/071954

(56) References cited:
- EP-A- 0 434 088
- DE-A1- 3 635 864
- US-A- 4 968 517
- US-A- 5 560 928

## Description

### Background of the invention

The invention presented herein refers to
effervescent tablets and effervescent granules
utilizing Carnitine as a Tartrate salt.

Carnitine is a natural product found in animal organisms and it is distributed to animal tissues. Pharmacological studies have demonstrated that only the levorotary enantiomer of Carnitine, namely L-Carnitine [as it will be referred to from here on wards (L-Carnitine=Levo-Carnitine), as it is customary in international bibliography]. The pharmacological effects of the molecule are associated to:
- The metabolism of lipids
- The metabolism of glucose
- The cardiovascular system, where L-Carnitine has .a definitive part in cardiac metabolism.

When L-Carnitine is administered per os, it is absorbed by the small intestine and the maximum plasma concentration is achieved rapidly at satisfactory levels. In the pharmaceutical market L-Carnitine may be found in the form of oral solutions, as free base or salt.

### Description of the invention

The present invention concerns effervescent formulations as defined in the table infra.

With the current invention, the production of effervescent tablets as well as effervescent granules, containing L-Carnitine Tartrate as an active ingredient, is achieved. Post effervescence, due to the presence of suitable and acceptable excipients a solution of L-Carnitine is delivered.

It is well known to the art that an effervescent formulation either in the form of tablets or granules yields a solution, that can be readily taken and which, in this respect, is more favorable to users by facilitating convenient transportation throughout the day, since dosing schedules feature more than one intakes per day, as opposed to an equivalent formulation of the active ingredient as an oral solution contained in vials.

### Qualitative and Quantitative composition of effervescent tablets and effervescent granules L-Carnitine Tartrate formulations

Effervescent tablets or effervescent granules

| Active Ingredient | |
|---|---|
| L-Carnitine Tartrate (equivalent to 1g of L-Carnitine) | 1.455,00 mg |

| Excipients | |
|---|---|
| Malic acid | 231,00 mg |
| Sorbitol | 1.950,00 mg |
| Citric acid anhydrous | 136,00 mg |
| Sodium bicarbonate anhydrous | 700,00 mg |
| Sodium carbonate anhydrous | 70,00 mg |
| Lemon essence (powder) | 130,00 mg |
| Acesulfame Potassium | 10,00 mg |
| Aspartame | 10,00 mg |
| Dibasic sodium citrate | 308,00 mg |

### Production Process

Effervescent tablets- Effervescent granules
1) The following are mixed: Sorbitol, Citric acid anhydrous, Malic acid.
2) In the mixture resulting from stage 1, L-Carnitine Tartrate is added and further mixture takes place.
3) The following are mixed: Sodium bicarbonate anhydrous, Sodium carbonate anhydrous, Dibasic sodium citrate and the resulting mixture is mixed with the mixture produced at stage 2.
4) In the mixture produced at stage 3, the following are added: Acesulfame Potassium, Aspartame, Lemon essence (powder) and further mixing takes place.
5) With the aforementioned method granules are produced which may be either packaged into sachets, in a low-humidity environment, or pressed into tablets, of 5-10kp hardness. These tablets are further packaged in aluminum stips.

## Claims

1. Effervescent formulations having the following composition:
L-Carnitine Tartrate (equivalent to 1 g of L-Carnitine) 1.455,00 mg, Malic acid 231,00 mg, Sorbitol 1.950,00 mg, Citric acid anhydrous 136,00 mg, Sodium bicarbonate anhydrous 700,00 mg, Sodium carbonate anhydrous 70,00 mg, Lemon essence (powder) 130,00 mg, Acesulfame Potassium 10,00 mg, Aspartame 10,00 mg, Dibasic sodium citrate 308,00 mg.

## Patentansprüche

1. Brauseformulierungen mit der folgenden Zusammensetzung:
L-Carnitintartrat (äquivalent zu 1 g von L-Carnitin) 1.455,00 mg, Apfelsäure 231,00 mg, Sorbitol 1.950,00 mg, wasserfreie Zitronensäure 136,00 mg, wasserfreies Natriumbicarbonat 700,00 mg, wasserfreies Natriumcarbonat 70,00 mg, Zitronenessenz (Pulver) 130,00 mg, Kalium Acesulfam 10,00 mg, Aspartam 10,00 mg, dibasisches Natriumcitrat 308,00 mg.

## Revendications

1. Formulations effervescentes ayant la composition suivante :
tartrate de L-carnitine (équivalent à 1 g de L-carnitine) 1 455,00 mg, acide malique 231,00 mg, sorbitol 1 950,00 mg, acide citrique anhydre 136,00 mg, bicarbonate de sodium anhydre 700,00 mg, carbonate de sodium anhydre 70,00 mg, essence de citron (poudre) 130,00 mg, acésulfame-potassium, 10,00 mg, aspartame 10,00 mg, citrate de sodium dibasique 308,00 mg.
